# EUROPÄISCHE PATENTANMELDUNG

(11) **EP 1 709 978 A1**
(43) Veröffentlichungstag der Anmeldung: **11.10.2006**
(21) Anmeldenummer: 05007557.1
(22) Anmeldetag: 06.04.2005
(51) Int. Cl.: A61L 2/18, A61L 2/16, A61L 2/24, A61B 19/00, A47L 15/00, C11D 1/00

(54) **Reinigung und Desinfektion chirurgischer und medizinischer Instrumente und Apparate**

(71) Anmelder: Chemische Fabrik Dr. Weigert GmbH & Co. KG., 20539 Hamburg (DE)
(72) Erfinder: Staffeldt, Jürgen, Dr., 21423 Winsen (DE); Wagemann, Wolfgang, Dr., 22967 Tremsbüttel (DE); Schmidt, Verona, 22605 Hamburg (DE)
(74) Vertreter: Glawe, Delfs, Moll

(57) **Zusammenfassung**

Gegenstand der Erfindung ist die Verwendung eines Reinigungsmittels das Tenside enthält und anwendungsfertig verdünnt in wässriger Lösung einen pH-Wert von wenigstens 10,5 aufweist, zur Inaktivierung von Mikroorganismen ausgewählt aus der Gruppe bestehend aus Bakterien, Viren und Pilzen bei der maschinellen Desinfektion von medizinischen und/oder chirurgischen Instrumenten und Apparaten.

## Beschreibung

Die Erfindung betrifft das Gebiet der Reinigung und Desinfektion medizinischer und/oder chirurgischer Instrumente und Apparate.

Medizinische und chirurgische Instrumente und Apparate müssen nach dem Gebrauch gereinigt und desinfiziert werden. Dies kann maschinell erfolgen. Die Desinfektion wird in der Regel getrennt von der Reinigung durchgeführt und beinhaltet entweder die Verwendung eines speziellen Mittels (chemische oder chemothermische Desinfektion), oder eine thermische Desinfektion.

Der Erfindung liegt die Aufgabe zugrunde, eine Möglichkeit zur Desinfektion medizinischer und/oder chirurgischer Instrumente und Apparate zu schaffen, bei der Bakterien, Viren und Pilze (Hefen und Schimmelpilze), insbesondere Bakterien und Pilze, auf wirtschaftlicher Art und Weise abgetötet bzw. inaktiviert werden. Die Erfindung soll zur routinemäßigen Anwendung bei der maschinellen Instrumentenreinigung und -aufbereitung geeignet sein.

Gegenstand der Erfindung ist daher die Verwendung eines Reinigungsmittels, das Tenside enthält und anwendungsfertig verdünnt in wässriger Lösung einen pH-Wert von wenigstens 11 aufweist, zur Abtötung/Inaktivierung von Mikroorganismen ausgewählt aus der Gruppe bestehend aus Bakterien, Viren und Pilzen, bei der maschinellen Reinigung und/oder Desinfektion von medizinischen und/oder chirurgischen Instrumenten und Apparaten.

Zunächst seien einige im Rahmen der Erfindung verwendete Begriffe erläutert.

Der Begriff Reinigungsmittel bezeichnet jede anwendungsfertige Formulierung, die entweder unmittelbar oder verdünnt mit Wasser zur Reinigung oder Desinfektion der entsprechenden Instrumente Verwendung findet. Im Kontext der Erfindung schließt der Begriff Reinigungsmittel den Begriff Desinfektionsmittel ein. Das Reinigungsmittel kann in fester Form oder vorzugsweise flüssig formuliert sein. Als Reinigungslösung, d.h. anwendungsfertig verdünnt in wässriger Lösung, weist das Reinigungsmittel einen pH-Wert von 10,5 oder größer auf.

Das erfindungsgemäß verwendete Reinigungsmittel enthält Tenside. Damit werden Verbindungen bezeichnet, welche die Grenzflächenspannung herabsetzen, also amphiphile Verbindungen mit mindestens einem hydrophoben und einem hydrophilen Molekülteil. Im Rahmen der Erfindung sind sämtliche Tenside wie beispielsweise anionische Tenside, nichtionische Tenside, kationische Tenside, amphotere Tenside und Blockcopolymere (insbesondere aus Ethylenoxid- und Propylenoxideinheiten) verwendbar. Beispielhaft verwiesen wird auf Römpp Chemielexikon, 10. Aufl., Stichwort "Tenside". Kationische Tenside können bevorzugt sein.

Die Erfindung findet Einsatz bei der maschinellen Reinigung und/oder Desinfektion medizinischer und oder chirurgischer Instrumente und/oder Apparate. "Maschinell" bedeutet, dass das Verfahren vorzugsweise in einer Spülmaschine automatisch abläuft und im Zuge der Reinigung bzw. der Desinfektion kein menschlicher Eingriff erforderlich ist. Insbesondere kann erfindungsgemäß in einer üblichen Spül- und Aufbereitungsmaschine für chirurgische Instrumente gearbeitet werden. Sie kann insbesondere zur routinemäßigen, täglichen Instrumentenreinigung verwendet werden.

Die Begriffe "Reinigung und/oder Desinfektion" beinhalten die erforderlichen Schritte bei der Aufarbeitung benutzter Instrumente und Apparate bis hin zu einem sauberen Zustand, an den sich vor der Wiederverwendung noch eine Sterilisierung anschließen kann.

Medizinische und/oder chirurgische Instrumente und Apparate sind sämtliche im medizinischen und Krankenhausbereich eingesetzten Geräte sowie Teile davon, die einer maschinellen Reinigung und Desinfektion grundsätzlich zugänglich sind.

Abtötung/Inaktivierung von Mikroorganismen bedeutet, dass diese hinreichend abgetötet werden, um eine gefahrlose anschließende bestimmungsgemäße Verwendung der chirurgischen Instrumente und Apparate zu ermöglichen. Die Anforderungen an chemische Desinfektionsmittel zur Prüfung der bakteriziden Wirkung sind in DIN EN 13727 und zur Prüfung der fungiziden Wirkung in DIN EN 13624 niedergelegt. Die viruzide Wirkung wird gemäß der Richtlinie des Bundesgesundheitsamtes und der Deutschen Vereinigung zur Bekämpfung von Viruskrankheiten e. V. geprüft.

Die Erfindung beruht auf der überraschenden Erkenntnis, dass eine im Stand der Technik lediglich als Reinigungsmittel eingesetzte Kombination von Alkalität und Tensiden eine hinreichende Wirkung gegen Bakterien, Viren und Pilzen bei der maschinellen Instrumentenreinigung und -aufbereitung aufweist. Es ist erfindungsgemäß insbesondere möglich, sowohl die Reinigung als auch die notwendige Desinfektion unter Verwendung eines einzigen Mittels durchzuführen, so dass die aufwendige und teure Vorratshaltung sowie Dosierung eines separaten Desinfektionsmittels unterbleiben kann. Ferner kann auf die energie- und zeitaufwändige sowie insbesondere für empfindliche Kunststoff- oder Gummiteile belastende Thermodesinfektion bei hohen Temperaturen verzichtet werden.

Der pH-Wert der anwendungsfertig verdünnten Reinigungslösung beträgt vorzugsweise wenigstens 11, weiter vorzugsweise wenigstens 11,5, weiter vorzugsweise wenigstens 12, weiter vorzugsweise wenigstens 12,5. Das Reinigungsmittel enthält bevorzugt Alkalihydroxide wie Natriumhydroxid oder bevorzugt Kaliumhydroxid. Die Verwendung von Kaliumhydroxid erleichtert das Bereitstellen eines Reinigungsmittels in Form eines Konzentrats, da Kaliumhydroxidlösungen bei niedrigen Temperaturen weniger zur Auskristallisation neigen als Natriumhydroxidlösungen. Das Reinigungsmittel kann zusätzlich Alkanolamine enthalten.

Durch Zusatz von Tensiden zu der hochalkalischen Reinigerlösung kann die Oberflächen- und Grenzflächenspannung deutlich reduziert werden. Grundsätzlich sind nichtionische Tenside wie bspw. Fettalkohole am besten zur Verminderung der Oberflächenspannung einer wässrigen Lösung geeignet. Sie haben den zusätzlichen Vorteil, dass sie wenig schäumen und somit die unerwünschte Schaumbildung bei der Reinigung medizinischer Instrumente verhindern oder vermindern. Eine Schaumbildung kann insbesondere den Umwälzpumpendruck in der Spülmaschine reduzieren, die Reinigung bspw. englumiger Schläuche von Endoskopen oder dergleichen beeinträchtigen. Nichtionische Tenside sind in stark alkalischen Milieu jedoch häufig schwierig in Lösung zu bringen. Es ist daher im Rahmen der Erfindung bevorzugt, das nichtionische Tensid mit kationischen, anionischen oder amphoteren Tensiden zu kombinieren, die für das nichtionische Tensid als Lösungsvermittler wirken können.

Die anwendungsfertig verdünnte Reinigerlösung weist vorzugsweise eine Oberflächenspannung von weniger als 50mN/m, vorzugsweise weniger als 40mN/m, weiter vorzugsweise weniger als 35 mN/m auf. Die Oberflächenspannung wird bestimmt nach der sogenannten Bügel-Ring-Methode nach DIN 53993.

Ein weiterer Aspekt der Erfindung ist die Vermeidung oder Verminderung der sogenannten Redeposition von Verunreinigungen auf den Instrumenten. Der Begriff Redeposition bezeichnet die Wiederablagerung einer bereits von einer kontaminierten Oberfläche entfernten Verunreinigung auf einer anderen, möglicherweise vorher nicht kontaminierten Oberfläche des zu reinigenden Instruments.

Bereits die im Rahmen der Erfindung vorgesehene Verwendung von Tensiden verhindert die Redeposition, da die Tenside abgelöste Verunreinigungen emulgieren können und damit in der wässrigen Lösung in Schwebe halten. Besonders bevorzugt ist im Rahmen der Erfindung zur Vermeidung oder Verringerung der Redeposition, dass das Reinigungsmittel zusätzlich Härtedispergatoren enthält. Als Härtedispergatoren können bspw. Phosphate und Polyphosphate, Komplex- oder Chelatbildner oder andere sogenannte Builder verwendet werden. Härtedispergatoren unterstützendie emulgierende Wirkung der Tenside und tragen somit zur Verhinderung der Redeposition bei.

Ein wichtiger Aspekt der Erfindung ist deren Eignung zur routinemäßigen, maschinellen Instrumentenreinigung und - aufbereitung. Für eine solche routinemäßige Reinigung werden im Stand der Technik üblicherweise schwachsaure oder schwachalkalische (bspw. enzymatische) Reiniger verwendet, da stark alkalische Lösungen zu einer erhöhten Beanspruchung oder Korrosion und damit Verschleiß von verschiedenen Materialien und Oberflächen führen können, die bei medizinischen Instrumenten und Apparaten verwendet werden. Problematisch in dieser Hinsicht sind bspw- Silikonelastomere, verchromte Instrumente, Lötverbindungen aus Silber und Zinn, Klebverbindungen und Dichtungsmaterialien, Kunststoffüberzüge wie bspw. Farbcodierungen, Glasfaserlichtleiter und optische Oberflächen mit Antireflexvergütung. Besonders problematisch sind Aluminiumoberflächen, insbesondere eloxierte Aluminiumoberflächen, da alkalische Lösungen diesen gegenüber eine besondere Aggressivität aufweisen. Die genannte Problematik tritt bspw. besonders auf bei der Reinigung von Endoskopen und deren Bestandteilen, da hier die zu reinigenden Oberflächen eine große Materialvielfalt aufweisen.

Bei einer bevorzugten Ausführungsform der Erfindung enthält daher das Reinigungsmittel zusätzlich Korrosionsinhibitoren. Darunter ist jeder Stoff zu verstehen, der in der alkalischen Lösung deren Angriff auf Oberflächen, insbesondere metallischen Oberflächen wie Aluminium oder eloxiertes Aluminium, hemmt. Geeignete Inhibitoren sind bspw. polymere Silicate wie bspw. Wasserglas, Phosphorsäureester oder dergleichen. Geeignete Phosphorsäureester sind Mono- und/oder Diester der Phosphorsäure mit aliphatischen Alkolholen der Kettenlänge C₁ bis C₂₂ und/oder aliphatischen Diolen und/oder aliphatischen Polyolen der Kettenlänge C₂ bis C₂₂. Erfindungsgemäß erhält man so trotz der Verwendung hochalkalischer Reinigerlösungen eine schonende Einwirkung auf bspw. eloxierte Aluminiumoberflächen.

Erfindungsgemäß wird aus den Bestandteilen des Reinigungsmittels vorzugsweise ein flüssiges Konzentrat formuliert, das mit Wasser zu der anwendungsfertigen Reinigungslösung verdünnt werden kann. In diesem Konzentrat liegt der Alkaligehalt (berechnet als KOH) vorzugsweise zwischen 2 und 30 Gew.-%, weiter vorzugsweise 15 und 26 Gew.-%. Der Tensidgehalt liegt bevorzugt zwischen 2 und 25 Gew.-%, weiter vorzugsweise 2 und 15 Gew.-%, weiter vorzugsweise 5 und 15 Gew.-%, weiter vorzugsweise 5 und 10 Gew.-%. Dieses Konzentrat wird vorzugsweise in Konzentrationen von 0,5 bis 5, vorzugsweise 0,5 bis 2, besonders bevorzugt 0,5 bis 1,5 Vol.-% mit Wasser zu einer gebrauchsfertigen Lösung angesetzt.

Wie bereits erwähnt, kann das Konzentrat wenigstens einen Komplexbildner, insbesondere Chelatbildner, enthalten. Die Komplexbildner dienen der Wasserenthärtung und können durch Komplexieren von Erdalkalionen die Reinigungswirkung gegenüber Kalkseifen verbessern. Bei den Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1-diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze.

Das Konzentrat kann Nitrilotriessigsäure und/oder ein Salz dieser Säure, besonders bevorzugt deren Trinatriumsalz, enthalten. Der NTA-Zusatz ist vorteilhaft, wenn das Konzentrat mit stark mineralstoffhaltigem (hartem) Wasser zu einer gebrauchsfertigen Lösung angesetzt werden soll.

Dem Konzentrat können übliche Konservierungsmittel zugesetzt werden, bspw. p-Hydroxybenzoesäure oder deren Methylester, 5-Brom-5-Nitro-1,3-dioxan, Salicylsäure, 2-Naphtylm-N-Di-methylthiocarbanilat, 5-Chlor-5-methyl-4-isothiazolin-3-on, 2-Methyl-4-isothiazolin-3-on sowie Gemische der beiden letztgenannten Verbindungen. Ein bevorzugtes Konservierungsmittel ist p-Hydroxybenzoesäure bzw. deren Methylester. Mit Hilfe dieser Konservierungsstoffe lässt sich Mikroben- und Pilzbefall des Reinigungsmittelkonzentrats vermeiden.

Bei Bedarf können Konfektionierhilfsmittel (Lösungsvermittler) zugegeben werden wie bspw. Natriumcumolsulfonat, Natriumtoluolsulfonat, Natriumxylolosulfonat, Harnstoff, Glykole, insbesondere Polypropylenglykole und Polyethylenglykole, Methylacetamid und Fettalkohole, wie bspw. Cetylalkohol.

Die Aufzählung möglicher Inhaltsstoffe ist nicht abschließend. Es können zusätzlich bspw. Netzmittel, Emulgatoren, schaumbremsende Mittel oder dergleichen zugesetzt werden. Vorteilhaft ist beispielsweise der Zusatz von N-Acylglutamat als Netzmittel.

Die Einwirkzeit des Reinigungsmittels beträgt erfindungsgemäß vorzugsweise 1 bis 60 min, weiter vorzugsweise 1 bis 30 min, weiter vorzugsweise 5 bis 30 min, weiter vorzugsweise 10 bis 20 min. Vor und/oder nach der Einwirkung des erfindungsgemäß verwendeten Reinigungsmittels können weitere Vorreinigungs-, Reinigungs-, Nachspül- oder Klarspül- oder Desinfektionsschritte vorgesehen werden. Bevorzugt ist es, zunächst ein Vorspülen zur Entfernung grober Verunreinigungen vorzunehmen, dann eine erfindungsgemäße Reinigung/Desinfektion, gefolgt von einem Nachspülen mit Wasser zur Entfernung von Reinigungsmittelresten.

Die Reinigung wird erfindungsgemäß bevorzugt bei einer Temperatur von Raumtemperatur bis 93°C, weiter vorzugsweise 40 bis 93°C, weiter vorzugsweise 50 bis 80°C, besonders bevorzugt 50 bis 60°C durchgeführt. Ebenfalls bevorzugt ist ein Temperaturbereich von Raumtemperatur (18°C) bis 50°C bzw. Raumtemperatur bis 40°C.

Bei der maschinellen Reinigung sind besonders bevorzugt Temperaturen von 50 bis 60°C, insbesondere etwa 55°C, und eine Einwirkzeit von 10 bis 20 min, vorzugsweise etwa 10 min. Das Reinigerkonzentrat gemäß dem nachfolgenden Beispiel 1 wird im Rahmen der maschinellen Reinigung bevorzugt in einer Anwendungskonzentration von etwa 1 bis 2 Vol.-% eingesetzt.

Gegenstand der Erfindung ist ferner ein Verfahren zur maschinellen Reinigung und/oder Desinfektion von medizinischen und/oder chirurgischen Instrumenten und Apparaten, bei dem in wenigstens einem Reinigungs- und/oder Desinfektionsschritt (nachfolgend und in den Patentansprüchen mit a) bezeichnet) eine Abtötung/Inaktivierung der genannten Mikroorganismen durch ein Reinigungsmittel, das Tenside enthält und anwendungsfertig verdünnt in wässriger Lösung ein pH-Wert von wenigstens 10,5 aufweist, erfolgt. Dieser Reinigungs- bzw. Desinfektionsschritt a) kann der erste Schritt des erfindungsgemäßen Verfahrens sein. Die verunreinigten Instrumente werden also ohne einen vorgeschalteten Reinigungsschritt unter sog. "dirty conditions" gereinigt und gleichzeitig desinfiziert. Diese Vorgehensweise hat den besonderen Vorteil, dass aus der Spülmaschine keinerlei kontaminiertes Abwasser abgelassen wird. Wenn dagegen in dem ersten oder den ersten Schritten des Verfahrens lediglich eine Reinigung ohne Inaktivierung der Mikroorganismen erfolgt, tritt kontaminiertes Abwasser aus der Spülmaschine aus und muss ggf. nachbehandelt werden, bevor es in die Kanalisation abgelassen werden kann. Bei der Inaktivierung der Mikroorganismen unter dirty conditions kann sich an diesen ersten Schritt a) beispielsweise ein Nachspülen und anschließendes Trocknen anschließen.

Erfindungsgemäß ist es ferner möglich, dass dem Reinigungs- bzw. Desinfektionsschritt a) ein oder mehrere weitere Vorspül- und/oder Reinigungsschritte vorgeschaltet sind. In diesem Fall wird in den ersten Schritten zunächst gereinigt, bevor dann unter sog. clean conditions die erfindungsgemäße Desinfektion erfolgt. Es ist besonders bevorzugt, wenn in dem vorgeschalteten Reinigungsschritt des gleiche Reinigungsmittel zur Herstellung der clean conditions verwendet wird wie in dem nachgelagerten Reinigungs- bzw. Desinfektionsschritt a). Es kann insbesondere in einer niedrigeren Konzentration (bevorzugt wenigstens um die Hälfte niedriger) verwendet werden, so dass in dem vorgelagerten Reinigungsschritt bei der niedrigeren Konzentration im wesentlichen gereinigt und erst in dem nachgelagerten Schritt erfindungsgemäß eine Abtötung/Inaktivierung der Mikroorganismen erfolgt. Besonders bevorzugte Parameter für den Reinigungs- bzw. Desinfektionsschritt a) sind Einwirktemperaturen von etwa 50 bis 60°C, insbesondere etwa 55°C, eine Einwirkzeit von 5 bis 15 Minuten, insbesondere etwa 10 Minuten, sowie eine Alkalikonzentration (berechnet als KOH) in der anwendungsfertig verdünnten Lösung von 0,05 bis etwa 0,2 Gew.-%, insbesondere etwa 0,1 Gew.-%. Die Tensidkonzentration in der anwendungsfertig verdünnten Lösung kann gering sein und bspw. bei etwa 0,01% bis 0,02 Gew.-% liegen. Die genannten Parameter stellen sich ein, wenn man ein Reinigungsmittelkonzentrat gemäß dem nachfolgenden Beispiel 1 im Verhältnis 1 zu 100 in Wasser löst.

Die erfindungsgemäße Verwendung bzw. das Verfahren ermöglichen eine wirksame Desinfizierung (Abtötung/Inaktivierung der genannten Mikroorganismen) von medizinischen und chirurgischen Instrumenten ohne den Einsatz in Stand der Technik üblicher separater Desinfektionswirkstoffe wie beispielsweise Aktivchlor, Peroxiden, Aminwirkstoffen oder dergleichen. Es ist somit auch Gegenstand der Erfindung, dass das erfindungsgemäß eingesetzte Reinigungsmittel solche herkömmlichen Desinfektionswirkstoffe nicht enthält.

### Beispiel 1

Ein Reinigungsmittelkonzentrat wird gemäß der nachfolgenden Tabelle zubereitet. Die Mengen der einzusetzenden Ausgangsstoffe sind in Gewichtsteilen angegeben. (Rest auf 100 Gewichtsteile Wasser)

| | |
|---|---|
| Kaliumtripolyphosphat | 21,39 |
| Kaliumhydroxid | 10,04 |
| Natriumalkylaminodipropionat | 2,40 |
| Fettalkohol, C10/12, 4EO, 4-5PO¹ | 0,50 |
| Natriumwasserglas | 27,90 |

| | |
|---|---|
| ¹Block-Co-Polymer aus C10/C12-Fettalkoholen mit 4 Ethylenoxid- und 4-5 Propylenoxideinheiten. | |

### Vergleichsbeispiel 1

Ein nicht erfindungsgemäßes Reinigungsmittelkonzentrat wird gemäß der nachfolgenden Tabelle zubereitet.

Es enthält bis auf die Tenside die gleichen Inhaltsstoffe wie das Konzentrat des Beispiels 1. (Rest auf 100 Gewichtsteile Wasser)

| | |
|---|---|
| Kaliumtripolyphosphat, 50% | 23,00 |
| Kaliumhydroxid, 45% | 10,80 |
| Natriumwasserglas | 30,00 |

### Beispiel 2

Die Reinigungsmittelkonzentrate gemäß Beispiel 1 und Vergleichsbeispiel 1 werden auf bakterizide Wirksamkeit gemäß DIN EN 13727:2003 und auf fungizide Wirksamkeit gemäß DIN EN 13624:2003 geprüft. Die genannten Normen und insbesondere die dort beschriebenen Bedingungen und Verfahren zur Prüfung der Wirksamkeit werden durch Bezugnahme darauf ausdrücklich zum Gegenstand auch der vorliegenden Anmeldung gemacht. Bei der Prüfung der Abtötung/Inaktivierung von Bakterien wurde der,Reduktionsfaktor der in der Norm genannten Mikroorganismen (siehe Tabelle 1) gemessen einer 1%igen wässrigen Lösung der Konzentrate gemäß Beispiel 1 bzw. Vergleichsbeispiel 1 bei einer Einwirkdauer von 5 min und einer Temperatur von 55°C. Diese Konzentration, Einwirkdauer und Temperatur entsprechen in etwa den typischen Bedingungen bei einer maschinellen Reinigung.

**Tabelle 1**

| | **geringe Belastung** | | | **hohe Belastung** | | |
|---|---|---|---|---|---|---|
| | **Ps. Aeruginosa** | **St. aureus** | **E. hirae** | **Ps. aeruginosa** | **St. aureus** | **E. hirae** |
| Vergleichsbeispiel 1 | >1,1E+05 | <9,6E+03 | <8,6E+03 | >1,1E+05 | <9,6E+03 | <8,6E+03 |
| Beispiel 1 | >1,1E+05 | >1,9E+05 | >1,7E+05 | >1,1E+05 | >1,9E+05 | >1,7E+05 |

In der Tabelle bedeutet "geringe Belastung", dass die Eiweißbelastung der verwendeten Prüfkörper gering ist. Dies entspricht der Inaktivierung der Mikroorganismen unter sog. clean conditions, bei denen vor der Desinfektion ein separater Reinigungsschritt vorgenommen worden ist. Entsprechend bedeutet "hohe Belastungen" eine hohe Belastung mit Eiweißrückständen, dies entspricht der Desinfektion (und gleichzeitigen Reinigung) unter sog. dirty conditions, bei denen keine vorgeschaltete separate Reinigung oder Vorspülung erfolgt.

Der Tabelle ist zu entnehmen, dass lediglich die Rezeptur gemäß Beispiel 1 die von der DIN EN 13727 geforderte Reduktion der Mikroorganismen Staphylococcus aureus und Enterococcus hirae um wenigstens den Faktor 10⁵ (5 log-Stufen) erreichen kann. Die alkalische Rezeptur gemäß Vergleichsbeispiel 1 erbringt diese Inaktivierungsleistung nicht. Man erkennt aus den Tabellen ferner, dass die Rezeptur gemäß Beispiel 1 erfindungsgemäß sowohl unter clean als auch unter dirty conditions die geforderte Inaktivierungsleistung erbringt.

In der nachfolgenden Tabelle 2 wird die fungizide Wirkung dargestellt, angegeben ist der Reduktionsfaktor einer 2%igen wässrigen Lösung bei einer Einwirkzeit von 10 min und einer Temperatur von 55°C.

**Tabelle 2**

| | **geringe Belastung** | | **hohe Belastung** | |
|---|---|---|---|---|
| | **C. albicans** | **A. niger** | **C. albicans** | **A. niger** |
| Vergleichsbeispiel 1 | 3,6E+03 | 1,8E+03 | 3,4E+03 | 2,0E+03 |
| Beispiel 1 | >1,8E+04 | >1,6E+04 | >1,4E+04 | >1,9E+04 |

Aus der Tabelle ist ersichtlich, dass lediglich Beispiel 1 die geforderte Inaktivierung um wenigstens den Faktor 10⁴ der Testfungi candida albicans und Aspergillus niger bewirkt.

### Beispiel 3

In diesem Beispiel werden zwei Programmabläufe zur Reinigung und Desinfektion von medizinischen und chirurgischen Instrumenten in einer üblichen Eintankspülmaschine angegeben.

Programmablauf 1:
- Vorreinigung mit Kaltwasser, 3 min,
- Reinigung mit einer 0,3 Vol.-%igen wässrigen Lösung der Rezeptur gemäß Beispiel 1 bei 55°C, Haltezeit 3 min,
- Reinigung/Desinfektion mit einer 1 Vol.-%igen wässrigen Lösung der Rezeptur gemäß Beispiel 1 bei 55°C, Haltezeit 10 min,
- Nachspülung mit Wasser unter Zudosierung eines sauren Neutralisationsmittels auf Basis Zitronensäure,
- Zwischenspülung mit Kaltwasser,
- Schlussspülung mit Wasser, Aufheizen auf 55°C mit Einwirkzeit von 1 min,
- Trocknung mit Heißluft.

Bei diesem Verfahrensablauf wird die Desinfektion unter sog. clean conditions durchgeführt, d.h. vor der eigentlichen Desinfektion wird zunächst eine Reinigung durchgeführt mit einer geringeren Konzentration des Reinigungsmittels gemäß Beispiel 1. Die im Programmablauf vorgesehene Kaltwasservorspülung kann optional ganz weggelassen werden. Die Nachspülung nach dem Desinfektionsschritt kann wahlweise auch ohne Neutralisationsmittel erfolgen, wenn hinreichend Wasser zum Abspülen der alkalischen Rezeptur gemäß Beispiel 1 eingesetzt wird.

Programmablauf 2:
- Vorreinigung mit Kaltwasser, 3 min,
- Reinigung/Desinfektion mit einer 1 Vol.-%igen wässrigen Lösung der Rezeptur gemäß Beispiel 1 bei 55°C, Haltezeit 10 min,
- Nachspülung mit Wasser unter Zudosierung eines sauren Neutralisationsmittels auf Basis Zitronensäure,
- Zwischenspülung mit Kaltwasser,
- Schlussspülung mit Wasser, Aufheizen auf 55°C mit Einwirkzeit von 1 min,
- Trocknung mit Heißluft.

Bei diesem Programmablauf erfolgt die Desinfektion unter dirty conditions, d.h. Reinigung und Desinfektion erfolgen in einem einzigen Schritt gleichzeitig. Optional kann die Kaltwasservorreinigung wegfallen. Dies hat bei der Reinigung unter dirty conditions den besonderen Vorteil, dass dann sämtliches aus der Spülmaschine austretendes Spülwasser nicht kontaminiert ist, da bereits die erste mit den verunreinigten Instrumenten in Berührung tretende Reinigerlösung desinfizierend wirkt und so Kontaminationen durch Mikroorganismen beseitigt.

Erfindungsgemäß ist eine abschließende Thermodesinfektion in keinem der Programmabläufe erforderlich. Dies verkürzt die Programmabläufe, da ein Aufheizen auf die Thermodisinfektionstemperatur von bspw. 93°C Zeit kostet, ferner wird Energie eingespart sowie der Verschleiß empfindlicher Instrumente (insbesondere Kunststoff- und Gummiteile) durch eine thermische Belastung vermindert.

### Beispiel 4

Zur Prüfung der Materialschonung eloxierter Aluminiumoberflächen werden eloxierte Aluminiumplatten in der Spülmaschine G7736 der Firma Miele für 10 min bei 55°C einem Reinigungsmedium ausgesetzt. Es werden sowohl neue farblos als auch blau eloxierte Aluminiumplatten verwendet. Das Reinigungsmedium ist 0,1 M NaOH mit einem pH-Wert von 12,7 bzw. eine 1 Vol.-%ige Reinigerlösung des Reinigungsmittelkonzentrats gemäß Beispiel 1. Anschließend werden die Platten visuell untersucht. Bei den mit NaOH behandelten Platten ist die Eloxalschicht deutlich abgetragen. Im Unterschied dazu weisen die mit dem Reiniger behandelten Platten keine sichtbaren Schädigungen der Eloxalschicht auf.

## Patentansprüche

1. Verwendung eines Reinigungsmittels, das Tenside enthält und anwendungsfertig verdünnt in wässriger Lösung einen pH-Wert von wenigstens 10,5 aufweist, zur Abtötung/Inaktivierung von Mikroorganismen ausgewählt aus der Gruppe bestehend aus Bakterien, Viren und Pilzen bei der maschinellen Desinfektion von medizinischen und/oder chirurgischen Instrumenten und Apparaten.

2. Verwendung nach Anspruch 1, **dadurch gekennzeichnet, dass** der pH-Wert wenigstens 11, vorzugsweise wenigstens 11,5, vorzugsweise wenigstens 12, weiter vorzugsweise wenigstens 12,5 beträgt.

3. Verwendung nach einem der Ansprüche 1 bis 2, **dadurch gekennzeichnet, dass** das Reinigungsmittel Alkalihydroxide enthält.

4. Verwendung nach Anspruch 3, **dadurch gekennzeichnet, dass** als Alkalihydroxid KOH verwendet wird.

5. Verwendung nach einem der Ansprüche 1 bis 4, **dadurch gekennzeichnet, dass** das Reinigungsmittel Alkanolamine enthält.

6. Verwendung nach einem der Ansprüche 1 bis 5, **dadurch gekennzeichnet, dass** das Reinigungsmittel nichtionische Tenside enthält.

7. Verwendung nach einem der Ansprüche 1 bis 6, **dadurch gekennzeichnet, dass** das Reinigungsmittel anwendungsfertig verdünnt eine Oberflächenspannung von weniger als 50 mN/m, vorzugsweise weniger als 40 mN/m, weiter vorzugsweise weniger als 35 mN/m aufweist.

8. Verwendung nach einem der Ansprüche 1 bis 5, **dadurch gekennzeichnet, dass** das Reinigungsmittel Härtedispergiermittel enthält.

9. Verwendung nach Anspruch 8, **dadurch gekennzeichnet, dass** das Reinigungsmittel Phosphate und/oder Polyphosphate enthält.

10. Verwendung nach einem der Ansprüche 1 bis 7, **dadurch gekennzeichnet, dass** das Reinigungsmittel Korrosionsinhibitoren enthält.

11. Verwendung nach Anspruch 10, **dadurch gekennzeichnet, dass** die Korrosionsinhibitoren ausgewählt sind aus der Gruppe besteht aus polymeren Silicaten und Phosphorsäureestern.

12. Verwendung nach einem der Ansprüche 1 bis 11, **dadurch gekennzeichnet, dass** die Einwirkzeit des Reinigungsmittels 1 bis 60 min, vorzugsweise 1 bis 30 min, weiter vorzugsweise 5 bis 30 min, weiter vorzugsweise 10 bis 20 min beträgt.

13. Verwendung nach einem der Ansprüche 1 bis 12, **dadurch gekennzeichnet, dass** die Reinigung bei einer Temperatur von Raumtemperatur bis 93°C, vorzugsweise 40 bis 93°C, weiter vorzugsweise 50 bis 80°C, weiter vorzugsweise 50 bis 60°C stattfindet.

14. Verfahren zur maschinellen Reinigung und/oder Desinfektion von medizinischen und/oder chirurgischen Instrumenten und Apparaten, **dadurch gekennzeichnet, dass** in wenigstens einem Reinigungs- und/oder Desinfektionsschritt a) durch ein Reinigungsmittel, das Tenside enthält und anwendungsfertig verdünnt in wässriger Lösung einen pH-Wert von wenigstens 10,5 aufweist, eine Abtötung/Inaktivierung von Mikroorganismen ausgewählt aus der Gruppe bestehend aus Bakterien, Viren und Pilzen erfolgt .

15. Verfahren nach Anspruch 14, **dadurch gekennzeichnet, dass** der Reinigungs- und/oder Desinfektionsschritt a) der erste Schritt des Verfahrens ist.

16. Verfahren nach Anspruch 14, **dadurch gekennzeichnet, dass** dem Reinigungs- und/oder Desinfektionsschritt a) ein Reinigungsschritt vorgeschaltet ist.

17. Verfahren nach Anspruch 16, **dadurch gekennzeichnet, dass** in dem vorgeschalteten Reinigungsschritt das gleiche Reinigungsmittel verwendet wird wie in dem Reinigungs- und/oder Desinfektionsschritt a).

18. Verfahren nach Anspruch 17, **dadurch gekennzeichnet, dass** in dem vorgeschalteten Reinigungsschritt die Konzentration des Reinigungsmittels niedriger ist als in dem Reinigungs- und/oder Desinfektionsschritt a).

19. Verfahren nach Anspruch 18, **dadurch gekennzeichnet, dass** in dem vorgeschalteten Reinigungsschritt die Konzentration des Reinigungsmittels um wenigstens die Hälfte niedriger ist als in dem Reinigungs- und/oder Desinfektionsschritt a).

20. Verfahren nach einem der Ansprüche 14 bis 19, **dadurch gekennzeichnet, dass** es keinen Thermodesinfektionsschritt umfasst.
